**Europäisches Patentamt**

**European Patent Office**

**Office européen des brevets**

(19)

(11) Veröffentlichungsnummer: **0 249 846**
**B1**

(12)

# EUROPÄISCHE PATENTSCHRIFT

(45) Veröffentlichungstag der Patentschrift:
**24.10.90**

(51) Int. Cl.⁵: **C07C 303/32**

(21) Anmeldenummer: **87108260.8**

(22) Anmeldetag: **06.06.87**

(54) **Verfahren zur Herstellung von festen Alkalimetallsalzen von alpha-Sulfofettsäurealkylestern.**

(30) Priorität: **14.06.86 DE 3620158**

(43) Veröffentlichungstag der Anmeldung:
**23.12.87 Patentblatt 87/52**

(45) Bekanntmachung des Hinweises auf die Patenterteilung:
**24.10.90 Patentblatt 90/43**

(84) Benannte Vertragsstaaten:
**AT BE CH DE ES FR GB IT LI NL SE**

(56) Entgegenhaltungen:
**AT-B- 173 690**
**DE-A- 3 319 591**
**FR-B- 1 254 678**
**US-A- 3 354 187**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Richtler, Hans-Joachim, Wiesenstrasse 16, D-4050 Mönchengladbach 4(DE)**
Erfinder: **Kreutzer, Udo, Dr., Robert-Koch-Strasse 1, D-4019 Monheim(DE)**
Erfinder: **Carduck, Franz-Josef, Dr., Landstrasse 18, D-5657 Haan(DE)**
Erfinder: **Köster, Klaus, Dr., Kurt-Schumacher-Strasse 1, D-4018 Langenfeld(DE)**
Erfinder: **Harth, Hubert, Dr., Buchenstrasse 31, D-4000 Düsseldorf(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von festen Alkalimetallsalzen von $\alpha$-Sulfofettsäurealkylestern mit einem Wassergehalt von weniger als 10 %, die sich als Detergentien in Wasch- und Reinigungsmittel eignen, durch gleichzeitige Bleichung und Neutralisation.

Die Herstellung von Alkalimetallsalzen von $\alpha$-Sulfofettsäurealkylestern ("Estersulfonaten") durch Sulfierung von Fettsäuremethylestern mit gasförmigem $SO_3$ und anschließende Neutralisation der entstandenen Sulfonsäuren mit wässrigen Alkalimetallhydroxiden, beispielsweise mit Natronlauge, ist seit langer Zeit bekannt. Die Verfahrensprodukte, also die Alkalimetallsalze der entsprechenden $\alpha$-Sulfofettsäurealkylester, finden überwiegend Einsatz als Detergentien in Wasch- und Reinigungsmitteln.

Alle bisher bekannten Herstellungsverfahren sind jedoch unbefriedigend geblieben, da entweder für die unmittelbare Verwendung in der Waschmittelindustrie geeignete hellfarbige Endprodukte gewonnen werden können, die Ausbeuten des dahin führenden Sulfonierungsschrittes jedoch sehr unbefriedigend sind, oder hohe Sulfonierungsausbeuten erreicht werden können, infolge der Farbinstabilität der Fettsäuren bzw. Fettsäureester in der Sulfonierungsstufe jedoch regelmäßig dunkel gefärbte bis braunschwarze Rohprodukte erhalten werden, die für eine unmittelbare Verwendung in Wasch- oder Reinigungsmitteln nicht geeignet sind. Einem zu hohen Produktausbeuten getriebenen Sulfonierungsschritt muß deswegen normalerweise eine Bleichung der im Sulfonierungsschritt angefallenen dunklen -Sulfofettsäurederivate angeschlossen werden, um für die Verwendung in Wasch- und Reinigungsmitteln geeignete, hellfarbige Produkte zu erhalten.

Eine weitere Schwierigkeit der bisher bekannten Verfahren zur Herstellung wässriger Estersulfonate besteht darin, daß die Produkte im Verlauf des Herstellungsverfahrens in Form Wasser enthaltender Pasten mit einem Wirkstoffgehalt bis zu 70 % anfallen. Derartige Pasten sind jedoch oberhalb von Konzentrationen von 30 % bis 40 % Estersulfonatgehalt ohne weitere Zusätze nicht mehr pumpbar. Das Viskositätsverhalten derartiger Estersulfonatpasten bringt also immer die Gefahr eines Verstopfens der Apparaturen und Leitungen mit sich. Es bestand daher seit langer Zeit ein Bedarf, Herstellungsverfahren für Alkalimetallsalze von $\alpha$-Sulfofettsäurealkylestern zu finden, in denen die hohe Viskosität der erhaltenen Produkte für die Verfahrensführung unschädlich ist.

In der JP-OS 8 416 870 wird ein Verfahren zur Herstellung von $\alpha$-Sulfofettsäureestersalzen beschrieben, in dem der im Verlauf des Sulfierschrittes enstandene $\alpha$-Sulfofettsäureester in Gegenwart einer Polycarbonsäure oder eines ihrer Salze mit $H_2O_2$ gebleicht und anschließend durch Neutralisation in das entsprechende $\alpha$-Sulfofettsäureestersalz überführt wird. Die dabei entstehenden Salze sind zwar zur Verwendung als Detergentien in Wasch- und Reinigungsmitteln sehr gut geeignet, die erforderliche Reaktionszeit für die Bleichung liegt jedoch in der Größenordnung von einer Stunde, in der die Zeit für die nachfolgende Neutralisationsreaktion nicht eingerechnet ist.

In der DE-OS 3 319 591 wird ein Verfahren zur Gewinnung farbstabiler, hellfarbiger wässriger Salzpasten waschaktiver $\alpha$-Sulfofettsäureester beschrieben, in dem die aus der Sulfierstufe stammenden dunkelfarbigen $\alpha$-Sulfofettsäureester zunächst in neutraler bis schwach alkalischer wässriger Lösung mit Alkalimetallhyperchloriten vorgebleicht und anschließend in schwach saurer wässriger Lösung - wie üblich - mit Wasserstoffperoxid nachgebleicht werden. Der gesamte Bleichvorgang dauert dabei mehrere Stunden, erfordert einen Wechsel des pH-Wertes des Reaktionsmediums mit allen damit zusammenhängenden Nachteilen und liefert wässrige Salzpasten, deren Wirkstoffgehalt im Bereich von 20 bis 60 Gew.-% liegt, was - wie oben beschrieben - aufgrund des starken Viskositätsanstiegs mit steigendem Aktivsubstanz-Gehalt zu verfahrenstechnischen Problemen führt.

Überraschend wurde nun gefunden, daß nahezu wasserfreie, feste $\alpha$-Sulfofettsäurealkylestersalze mit hervorragenden Farbwerten und hoher Farbstabilität dadurch erhalten werden können, daß man die Bleichung und Neutralisation der aus dem Sulfierschritt resultierenden, dunkelfarbigen $\alpha$-Sulfofettsäurealkylester in einem Verfahrensschritt durchführt.

Die Erfindung betrifft somit ein Verfahren zur Herstellung von festen, nahezu wasserfreien Alkalimetallsalzen von $\alpha$-Sulfofettsäurealkylestern durch oxidative Bleichung und Neutralisation von $\alpha$-Sulfofettsäurealkylestern mit 8 bis 22 C-Atomen in der Fettsäurekette und 1 bis 6 C-Atomen im Ester-Alkylrest, das dadurch gekennzeichnet ist, daß man in einer zur Verarbeitung pastöser Produkte geeigneten Anlage die festen oder geschmolzenen $\alpha$-Sulfofettsäurealkylester bei Temperaturen im Bereich von 20 bis 80°C gleichzeitig mit einer wässrigen Lösung von Wasserstoffperoxid oder einer $H_2O_2$ liefernden Verbindung und festem Alkalimetallcarbonat, gegebenenfalls in Gegenwart von wässrigem Alkalimetallhydroxid, vermischt, wobei das Gewichtsverhältnis Ester : $H_2O_2$ im Bereich von 1 : 0,01 bis 0,06 und das Molverhältnis Ester : Alkalimetallcarbonat im Bereich von 1 : 0,5 bis 1 : 0,75 liegt, den durch Freiwerden von $CO_2$ entstehenden Schaum bei einem Druck von 0,2 bis 1,0 bar und einer Temperatur von 50 bis 70°C mechanisch zerstört, das entstehende Produkt bei einem Druck von 15 bis 100 mbar und einer Temperatur von 50 bis 80°C unter mechanischer Bewegung restentgast und restentwässert und die entstehenden Feststoffe nach bekannten Verfahren konfektioniert.

Zur Durchführung des erfindungsgemäßen Verfahrens können alle zur Verarbeitung pastöser Produkte geeigneten Apparate, Anlagen oder Aggregate eingesetzt werden, sofern sie so beschaffen sind, daß man in den entsprechenden Verfahrensschritten zur Entgasung unter Vakuum arbeiten und entstehenden Schaum mechanisch zerstören kann. Geeignet sind beispielsweise entsprechend ausgerüstete Rührkessel und Trogwär-

meaustauscher. Bevorzugt werden evakuierbare Kneter, beispielsweise Z-Kneter, eingesetzt. Wenn das erfindungsgemäße Verfahren in einer bevorzugten Ausführungsform in kontinuierlicher Arbeitsweise durchgeführt werden soll, kommen kontinuierlich arbeitende Fest-/Flüssigmischer zum Einsatz. Hierfür haben sich insbesondere Einspritzmischer oder nach dem Rotor-/Stator-Prinzip arbeitende Apparate als geeignet erwiesen.

Die als Ausgangsmaterialien des erfindungsgemäßen Verfahrens verwendeten α-Sulfofettsäurealkylester entstammen dem nach dem Stand der Technik üblichen Sulfierungsverfahren von Fettsäurealkylestern. Derartige Ester stammen aus synthetischen, halbsynthetischen oder natürlichen Ölen und/oder Fetten, wobei diese ihren Ursprung in Pflanzen, Land- oder Wassertieren haben können. Ihre Fettsäurereste haben eine Anzahl von C-Atomen im Bereich von 8 bis 22, wobei C-Zahlen im Bereich von 12 bis 18 C-Atomen bevorzugt sind. Dies sind also Laurinsäure, Tridecansäure, Myristinsäure, Pentadecansäure, Plamitinsäure, Margarinsäure oder Stearinsäure. Die Estergruppierung der Fettsäurealkylester weist normalerweise C-Zahlen im Bereich von 1 bis 6 auf, wobei C-Zahlen im Bereich von 1 bis 3 und insbesondere die entsprechenden Methylester bevorzugt sind. Diese entstehen im allgemeinen daraus, daß man synthetische, halbsynthetische oder natürliche Öle und/oder Fette und deren Gemische verseift und die entstehenden Fettsäuren mit einwertigen Alkoholen mit 1 bis 6 C-Atomen umsetzt oder auf direktem Wege durch Umsatz mit den entsprechenden Alkoholen, insbesondere Methanol, umestert. Die entsprechenden Fettsäureester werden anschließend bei erhöhter Temperatur auf an sich bekanntem Wege in einem Sulfierungsreaktor mit einem Gemisch von gasförmigem Schwefeltrioxid und Inertgas sulfoniert, wobei Produkte mit einem Sulfonierungsgrad von mehr als 90 % resultieren, die aufgrund der Farbinstabilität der Fettsäurealkylester mehr oder weniger dunkel gefärbt sind.

Entsprechend dem erfindungsgemäßen Verfahren lassen sich nun neutrale bis schwach alkalische α-Sulfofettsäurealkylester-Salze ("Estersulfonate") mit einem Wassergehalt von weniger als 10 % und Klett-Farbwerten kleiner als 100 durch gleichzeitige oxidative Bleichung und Neutralisation herstellen. Als Ausgangsstoffe werden dabei die Rohprodukte des Sulfierungsschrittes von Fettsäurealkylestern mit 8 bis 22 C-Atomen, bevorzugt mit 12 bis 18 C-Atomen, in der Festsäurekette, bevorzugt die α-Sulfofettsäurealkylester, eingesetzt.

Die Bleichung erfolgt dabei mit Wasserstoffperoxid oder Verbindungen, die Wasserstoffperoxid in wässriger Lösung unter den Reaktionsbedingungen freisetzen; die gleichzeitige Neutralisation erfolgt durch Alkalimetallcarbonate, insbesondere durch Natriumcarbonat. Im Zuge dieser gleichzeitigen Bleich- und Neutralisationsreaktion wird überraschenderweise die Bleichwirkung des Wasserstoffperoxids im Vergleich zu einer aus dem Stand der Technik bekannten zweistufigen Bleiche und Neutralisation deutlich verstärkt. Eine zweite alkalische Bleiche wird dadurch überflüssig. Als Produkte der gleichzeitigen Bleich- und Neutralisationsreaktion entstehen beim Abkühlen der Reaktionsmasse auf Raumtemperatur neutrale bis schwach alkalische Feststoffe, die nach an sich bekannten Methoden pastilliert, granuliert oder geschuppt werden können. Die Produkte sind nicht klebrig und lösen sich hervorragend in Wasser.

Die aus der Sulfierung von Fettsäurealkylestern stammenden, mehr oder weniger dunkel gefärbten α-Sulfofettsäurealkylester werden in das erfindungsgemäße Verfahren in nahezu wasserfreiem Zustand eingesetzt. Dabei können die Ausgangsstoffe fest oder geschmolzen sein, was von den als Rohstoff eingesetzten α-Sulfofettsäurealkylestern und der jeweils gewählten Reaktionstemperatur abhängig ist. Die Reaktionstemperatur liegt praktischerweise im Bereich von 20 bis 80°C, wobei der Bereich von 50 bis 80°C für die Durchführung des erfindungsgemäßen Bleich- und Neutralisationsverfahrens besonders bevorzugt ist. Bei hohen Temperaturen, beispielsweise im Bereich von 60 bis 80°C, liegen dann die eingesetzten α-Sulfofettsäurealkylester bevorzugt im geschmolzenen Zustand vor.

Die als Ausgangsstoffe eingesetzten α-Sulfofettsäurealkylester, von denen - wie oben beschrieben - die jeweiligen Methylester infolge ihrer leichten Zugänglichkeit aus nativen Quellen wie Talg, Kokosöl oder Palmkernöl nach Umsetzung mit Methanol besonders bevorzugt sind, werden im angegebenen Temperaturbereich gleichzeitig mit einer wässrigen Lösung von Wasserstoffperoxid oder einer Wasserstoffperoxid liefernden Verbindung und festem Alkalimetallcarbonat versetzt. In der Praxis werden bevorzugt Wasserstoffperoxidlösungen mit einem Gehalt von 30 bis 70 Gewichtsprozent $H_2O_2$ eingesetzt. Hierbei sind die für die Handhabung von konzentrierten Wasserstoffperoxidlösungen erforderlichen Sicherheitsvorkehrungen zu treffen. Die Konzentration von Wasserstoffperoxid liefernden Verbindungen wird so bemessen, daß die Lösungen eine Menge Wasserstoffperoxid zur Verfügung stellen, die dem oben angegebenen Konzentrationsbereich für $H_2O_2$ entspricht.

Das Gewichtsverhältnis von α-Sulfofettsäurealkylester : $H_2O_2$ ist dabei auf einen Bereich von 1 : 0,01 bis 0,06 und das Molverhältnis α-Sulfofettsäurealkylester : Alkalimetallcarbonat auf einen Bereich von 1 : 0,5 bis 1 : 0,75 einzustellen. Bevorzugt liegt das Gewichtsverhältnis Ester : $H_2O_2$ bei 1 : 0,03 bis 1 : 0,05 und das Molverhältnis Ester : Alkalimetallcarbonat im Bereich 1 : 0,5 bis 1 : 0,65.

Gemäß dem erfindungsgemäßen Verfahren werden der α-Sulfofettsäurealkylester bzw. die unmittelbar aus dem Sulfierschritt nativer Fettsäurealkylester resultierenden Estermischungen, die aufgrund ihrer leichten Zugänglichkeit bevorzugt als Ausgangsstoffe verwendet werden, in Substanz mit dem Bleichmittel und dem Neutralisationsmittel in einem geeigneten Mischer miteinander vermischt, wobei die Zugabe der wässrigen Bleichlösung und des Neutralisationsmittels portionsweise innerhalb kurzer Zeit erfolgt, um ein Überschaumen der Reaktionsmischung zu vermeiden. Die Reaktion setzt spontan ein; ihr Beginn kann dadurch erkannt werden, daß die Mischung durch freiwerdendes Kohlendioxid aufschäumt und sich im Laufe der Zeit aufhellt. Je nach eingesetzter Rohstoffart und -menge werden in dieser Reaktionsstufe (gemessen unter Normalbedingungen) 30 bis 80 l $CO_2$/kg eingesetzte Sulfonsäure frei.

In einer bevorzugten Ausführungsform des erfindungsgemäßen Verfahrens kann ein Teil der Sodamenge in der Bleich-/Neutralisationsmischung durch einen Zusatz von Alkalimetallhydroxid ersetzt werden. In diesem Fall wird eine Bleich-/Neutralisationsmischung mit den oben beschriebenen Mengen und Konzentrationen an Wasserstoffperoxid verwendet, in der das Molverhältnis Ester : Alkalimetallcarbonat im Bereich von 1 : 0,5 bis 1 : 0,525 und das Molverhältnis Ester : Alkalimetallhydroxid im Bereich von 1 : 0,03 bis 0,08 liegt. Dies bedeutet, daß (bezogen auf die Menge an α-Sulfofettsäurealkylester) ein geringer Überschuß (100 bzw. 105 % des stöchiometrischen Einsatzes) an Alkalimetallcarbonat und 3 bis 8 % einer wässrigen Alkalimetallhydroxidlösung verwendet werden. Bevorzugt wird für diesen Zweck eine wässrige Lösung von NaOH verwendet. Diese Lösung hat vorteilhafterweise eine Konzentration von 50 % NaOH.

In dem erfindungsgemäßen Verfahren können sowohl in Gegenwart als auch in Abwesenheit der wässrigen Alkalimetallhydroxidlösung als Alkalimetallcarbonat alle denkbaren Alkalimetallcarbonate einzeln oder in Mischungen miteinander verwendet werden. Aufgrund der preiswerten Verfügbarkeit wird jedoch bevorzugt Natriumcarbonat für das erfindungsgemäße Verfahren verwendet.

Beim Vermischen der α-Sulfofettsäurealkylester bzw. ihrer Mischungen mit dem Bleichmittel und dem Neutralisationsmittel (Peroxid und Alkalimetallcarbonat, gegebenenfalls in Gegenwart von Alkalimetallhydroxid) entsteht ein feinporiger, stabiler Schaum, der zur Herstellung der festen, aufgehellten α-Sulfofettsäurealkylestersalze nur noch entgast und entwässert werden muß. Dies geschieht erfindungsgemäß in zwei Stufen.

In der ersten Entgasungsstufe wird der durch Freiwerden von $CO_2$ entstehende α-Sulfofettsäurealkylestersalz-Schaum unter Atmosphärendurck oder leichtem Vakuum, d.h. in einem Druckbereich von 0,02 bis 1,0 bar mechanisch zerstört. Dies geschieht in einem als Entgasungsapparat geeigneten Rührkessel, Kneter, Trogwärmetauscher mit selbstreinigenden Misch- und Knetwällen oder einem ähnlichen, für diese Zwecke geeigneten Behälter. Dieser Behälter wird auf eine Temperatur von 50 bis 70°C thermostatisiert, um die freiwerdende Neutralisationswärme abzuführen. Die zur Entgasung in der ersten Stufe erforderliche Verweilzeit der Reaktionsmischung in dem Behälter beträgt dabei 5 bis 15 min. Dabei liegt die Verweilzeit im unteren Bereich dieser Zeitspanne, wenn die Entgasung - wie bevorzugt - bei einem Druck von 0,2 bis 0,5 bar durchgeführt wird. Im Verlaufe dieser Zeitspanne wird bereits ein großer Teil (ca. 60 bis 90 %) des entstandenen Kohlendioxids aus der Reaktionsmasse entfernt. Die Viskosität und Dichte der Masse steigt mit fortschreitender Entgasung an. Das Produkt wird dabei zunehmend klebriger.

In einer weiteren Stufe wird dann die Reaktionsmasse im Vakuum restentgast und restentwässert. Dabei wird ein Druck von 15 bis 100 mbar angelegt und die entsprechenden Entgasungsapparate auf einen Temperaturbereich von 50 bis 80°C thermostatisiert. Unter intensivem Kneten oder in einer mechanisch erzeugten dünnen Schicht, beispielsweise einem Trogwärmetauscher oder einem Dünnschichtverdampfer, wird die Restmenge an Kohlendioxid im Vakuum ausgetrieben und auch ein Großteil des mit dem Bleich- und dem Neutralisationsmittel eingeschleppten und auch des bei der Neutralisation im Zuge der Reaktion entstehenden Wassers mit abgezogen. Die Verweilzeit der Reaktionsmischung in dieser Restentgasungs- und Restentwässerungsstufe liegt ebenfalls im Minutenbereich. Als Produkte werden mit fortschreitender Entgasung wachs- oder seifenähnliche, nicht fließfähige Massen heller α-Sulfofettsäurealkylester erhalten, die in geschmolzenem Zustand eine Viskosität von über 1000 Pa.s aufweisen und bei Abkühlung auf Raumtemperatur erstarren.

Die auf diesem Wege erhaltenen Feststoffe können nun nach an sich bekannten Verfahren konfektioniert werden. Dies kann mit handelsüblichen Schneckenextrudern geschehen, so daß als Produkt dünne Stränge von -Sulfofettsäurealkylestern entstehen. Dabei sind keine weiteren Zuschlagsstoffe erforderlich. Es entstehen auf diesem Wege feste Produkte mit einem Waschaktivsubstanzgehalt von über 80 %.

Alle bei dem erfindungsgemäßen Verfahren erhaltenen Produkte sind nicht-klebrig und lösen sich hervorragend in Wasser. Sie unterscheiden sich damit in vorteilhafter Weise von größeren Mengen Wasser enthaltenden α-Sulfofettsäurealkylestern nach dem Stand der Technik, deren großtechnische Verarbeitung aufgrund des für diese Produkte eigenartigen Viskositätsverhaltens große Schwierigkeiten mit sich bringt.

Ein unmittelbarer Vorteil des erfindungsgemäßen Verfahrens ist darin zu sehen, daß die Gesamtreaktions- und Entgasungszeit vom Einsatz der dunklen, in α-Stellung sulfonierten Fettsäurealkylester bis zum Erhalt der hellen Reaktionsprodukte je nach Konzentration der Reaktionspartner und Farbwert der eingesetzten Ester im Bereich von 15 bis 45 min liegt und dabei deutlich kürzer ist, als dies für nach dem Stand der Technik bekannte, in allen Fällen mehrstufige Verfahren beschrieben wurde. Zudem entstehen Produkte mit einem extrem hohen Feststoff- bzw. Aktivsubstanzgehalt, deren Weiterverarbeitung bzw. Einsatz als Detergentien in Wasch- und Reinigungsmitteln sehr viel besser als bei Produkten aus dem Stand der Technik möglich ist. Aus rohen α-Sulfofettsäureestern durch Neutralisation und gegebenenfalls Bleichung hergestellte Alkalimetallsalze von α-Sulfofettsäureestern enthalten immer einen gewissen Anteil an Dialkalimetallsalzen der freien α-Sulfofettsäuren. Ein Teil dieser Disalze stammt aus der alkalischen Hydrolyse der im rohen Sulfonierungsprodukt anteilweise enthaltenen gemischten Anhydride aus α-Sulfofettsäureestern und Methylschwefelsäure. Ein weiterer Anteil an Disalz hat seinen Ursprung in der unerwünschten

alkalischen Hydrolyse des α-Sulfofettsäuremethylesters bei erhöhter Temperatur und bei pH-Werten von 9 und darüber. Überraschenderweise hat es sich gezeigt, daß die nach dem erfindungsgemäßen Verfahren hergestellten Alkalimetallsalze von α-Sulfofettsäureestern keinen höheren Disalzgehalt aufweisen, als solche, die nach den Verfahren des Standes der Technik hergestellt wurden, obwohl hier festes Alkalimetallcarbonat, gegebenenfalls in Kombination mit wässrigem Alkalimetallhydroxid als Neutralisationsmittel eingesetzt wird.

Die durch das erfindungsgemäße Verfahren erzielte Verbesserung der Farbwerte führt zu hellen Feststoffen, die sich ohne weitere Bleichung oder Reinigung für den Einsatz als Detergentien in Wasch- und Reinigungsmitteln eignen. Die erreichbaren, nach Klett bestimmten Farbzahlen liegen in allen Fällen des erfindungsgemäßen Verfahrens unter 100. Die erzielbaren Werte sind damit deutlich besser als in aus dem Stand der Technik bekannten, mehrstufigen Bleich- und Neutralisationsverfahren.

Die Erfindung wird durch die nachfolgenden Beispiele näher erläutert.

Beispiel 1

600 g geschmolzener α-Sulfofettsäuremethylester (Basis: Palmkernöl, Länge der Fettsäurekette: 12 bis 18 C-Atome, Säurezahl: 232, Klett-Farbzahl: 250) wurden in einem Z-Kneter (Firma Jahnke & Kunkel) durch Erwärmen auf 60°C geschmolzen. Dem Ester wurden aus getrennten Vorratsgefäßen 34,3 g 35 %ige wässrige Wasserstoffperoxidlösung und 158 g Natriumcarbonat innerhalb von 5 min zugegeben und damit vermischt; dabei wurde die Zugabe so geregelt, daß ein Überschäumen des Kneters vermieden wurde. Der Kneter wurde zur Abführung der Neutralisationswärme auf 50°C thermostabilisiert. Entstehender Schaum wurde durch die Knetermechanik zerstört.

Ester, Wasserstoffperoxid und Soda wurden 20 min unter Atmosphärendruck geknetet, wobei das Kohlendioxid . teilweise entwich und sich die Farbe der Mischung deutlich aufhellte.

Danach wurde das schaumige, klebrige Material bei 60°C 30 min im Vakuum entgast, wobei der Druck kontinuierlich von 1,0 bar auf 25 mbar reduziert wurde.

Es entstand eine Paste, die beim Erkalten auf Raumtemperatur erstarrte.

Analysewerte des Produktes:
pH-Wert einer 1 %igen Lösung: 6,9;
Farbzahl nach Klett: 35;
Wassergehalt: 1,4 %;
Disalz-Gehalt: 22 % (entsprechend dem Anhydridgehalt des eingesetzten Esters)
WAS-Gehalt: 89 %

Die Messung der Farbzahl nach Klett erfolgte in diesem wie auch den folgenden Beispielen mit einer 5 %igen wässrigen Lösung des Produktes in einer Küvette mit 4 cm Schichtdicke unter Verwendung eines Blaufilters bei 400 bis 465 nm.

Die Messung des pH-Wertes erfolgte in diesem wie auch den folgenden Beispielen in einer 1 %igen wässrigen Lösung des Produktes unter Verwendung einer Glaselektrode.

Die Bestimmung der Säurezahl und des Disalz-Gehaltes erfolgte in diesem wie auch den folgenden Beispielen durch potentiometrische Titration. Der Gehalt an waschaktiver Substanz (WAS) wurde durch Epton-Titration ermittelt.

Beispiel 2

Analog Beispiel 1 wurden 600 g des dort beschriebenen α-Sulfofettsäuremethylesters auf der Basis Palmkernöl verarbeitet. Neben der in Beispiel 1 beschriebenen Menge an Wasserstoffperoxid wurden hier jedoch 138,5 g Natriumcarbonat (entsprechend einem 5 %igen Überschuß über die stöchiometrische Menge) und 30 g einer 50 %igen wässrigen Natronlauge eingesetzt.

Die Behandlung der Reaktionsmischung erfolgte wie in Beispiel 1 angegeben. Es wurde eine feste Paste mit folgenden Analysenwerten erhalten:
pH-Wert einer 1 %igen Lösung: 8,2;
Klett-Farbzahl: 39;
Wassergehalt: 1,5 %;
Disalz-Gehalt: 23 %;
WAS-Gehalt: 84 %.

Beispiel 3

Die Herstellung des α-Sulfofettsäuremethylesters erfolgte entsprechend der in Beispiel 1 angegebenen Verfahrensvorschrift. Als Ausgangsprodukt wurde jedoch ein α-Sulfofettsäuremethylester auf der Basis von Talg eingesetzt, dessen Fettsäurerest Kohlenstoffzahlen im Bereich von 16 bis 18 aufwies (Säurezahl: 197; Klett-Farbzahl: 1000). Es wurden 600 g des α-Sulfofettsäuremethylesters, 84 g 35 %iger Wasserstoffperoxidlösung und 123 g Natriumcarbonat eingesetzt. Das Produkt hatte folgende Analysenwerte:

pH-Wert einer 1 %igen Lösung: 7,7;
Klett-Farbzahl: 70;
Wassergehalt: 3,3 %;
Disalz-Gehalt: 24 %;
WAS-Gehalt: 83 %.

Beispiel 4

In einem DTB-3b-Trogwärmetauscher (Hersteller Fa. List, Pratteln, CH) wurden 3 kg eines α-Sulfofettsäurealkylesters (Zahl der C-Atome in der Fettsäurekette: 12 bis 18; Säurezahl: 232; Klett-Farbzahl: 250) geschmolzen und mit 720 g Natriumcarbonat und 171 g einer 35 %igen wässrigen Wasserstoffperoxid-Lösung versetzt. Das stark schäumende, hellgelbe Produkt wurde 15 min bei 0,2 bar Druck entgast. Zur Restentgasung wurde der Druck auf 25 mbar reduziert. Während der Restentgasung wurde der Behälter auf 60°C thermostatisiert. Im Verlauf des 20-minütigen Entgasungsvorgangs wurden neben Kohlendioxid auch 150 ml H₂O abgezogen. Es entand ein gelblich-weißes, wachsartiges Produkt, das beim Erkalten auf Raumtemperatur zu einer brüchigen Masse erstarrte. Diese wurde in einem Buss-Ko Kneter zu dünnen Strängen extrudiert.

Anlaysenwerte des Produktes:

pH-Wert einer 1 %igen wässrigen Lösung: 6,8;
Klett-Farbzahl: 40;
Wassergehalt: 1,3 %;
Disalz-Gehalt: 20,5 %;
WAS-Gehalt: 83 %.

Beispiel 5

200 g eines α-Sulfofettsäuremethylesters auf Basis von Kokosöl (Säurezahl: 232; Klett-Farbzahl: 250) wurden mit 112 g $H_2O_2$ (35 %ige wässrige Lösung), 46,8 g Natriumcarbonat und 10 g 50%iger wässriger Natronlauge in einem Kneter 30 min bei 60°C gebleicht und neutralisiert. Das Produkt wurde anschließend im Vakuum entgast (Gesamtreaktionszeit: 60 min).

Das nahezu farblose Reaktionsprodukt hatte folgende Analysenwerte:

pH-Wert einer 1 %igen wässrigen Lösung: 6,9;
Klett-Farbzahl: 17;
Disalz-Gehalt: 19,5 %;
WAS-Gehalt: 85 %.

Beispiel 6

In einem Einspritzmischer (siehe Prospekt "Der FMC Kontinuierliche Hochleistungseinspritzmischer, FMC Food Machinery Europe 1983, printed in Belgium) wurden 50 kg/h einer Schmelze von α-Sulfofettsäuremethylester auf Basis Kokosöl kontinuierlich unter Kühlung mit 13,2 kg/h Natriumcarbonat und 4,3 kg/h einer 35 gew.-%igen wässrigen $H_2O_2$-Lösung vermischt. Der resultierende weiße Schaum wurde in einem Paddelschneckenapparat mechanisch zerstört und in Vakuum entgast und entwässert. Das nahezu farblose Reaktionsprodukt erstarrt unterhalb von 55 °C und wurde zu Pulver zerrieben. Folgende Analysenwerte wurden gefunden:

pH-Wert einer 1 %igen wässrigen Lösung: 6,9
Klett-Farbzahl 70
Wassergehalt 2,4 %
Disalz-Gehalt 22 %
WAS-Gehalt 92 %

## Patentansprüche

1. Verfahren zur Herstellung von festen, nahezu wasserfreien Alkalimetallsalzen von α-Sulfofettsäurealkylestern durch oxidative Bleichung und Neutralisation von α-Sulfofettsäurealkylestern mit 8 bis 22 C-Atomen in der Fettsäurekette und 1 bis 6 C-Atomen im Ester-Alkylrest, dadurch gekennzeichnet, daß man
    (a) in einer zur Verarbeitung pastöser Produkte geeigneten Anlage die festen oder geschmolzenen α-Sulfofettsäurealkylester bei Temperaturen im Bereich von 20 bis 80°C gleichzeitig mit einer wässrigen Lösung von Wasserstoffperoxid oder einer $H_2O_2$ liefernden Verbindung und festem Alkalimetallcarbonat, gegebenenfalls in Gegenwart von wässrigem Alkalimetallhydroxid, vermischt, wobei das Gewichtsverhältnis Ester : $H_2O_2$ im Bereich von 1 : 0,01 bis 0,06 und das Molverhältnis Ester : Alkalimetallcarbonat im Bereich von 1 : 0,5 bis 1 : 0,75 liegt,
    (b) den durch Freiwerden von $CO_2$ entstehenden Schaum bei einem Druck von 0,2 bis 1,0 bar und einer Temperatur von 50 bis 70°C mechanisch zerstört,
    (c) das entstehende Produkt bei einem Druck von 15 bis 100 mbar und einer Temperatur von 50 bis 80°C unter mechanischer Bewegung restentgast und restentwässert und
    (d) die entstehenden Feststoffe nach bekannten Verfahren konfektioniert.

2. Verfahren nach Anspruch I, dadurch gekennzeichnet, daß man als zur Verarbeitung pastöser Produkte geeignete Anlage einen Rührkessel, Kneter oder Schneckenmischer einsetzt.

3. Verfahren nach Ansprüchen 1 und 2, dadurch gekennzeichnet, daß man geschmolzene α-Sulfofettsäurealkylester einsetzt.

4. Verfahren nach Ansprüchen 1 und 2, dadurch gekennzeichnet, daß man α-Sulfofettsäurealkylester mit 12 bis 18 C-Atomen in der Fettsäurekette einsetzt.

5. Verfahren nach Ansprüchen 1 und 2, dadurch gekennzeichnet, daß man α-Sulfofettsäurealkylester mit 1 bis 3 C-Atomen im Esteralkylrest, bevorzugt α-Sulfofettsäuremethylester, einsetzt.

6. Verfahren nach Ansprüchen 1 bis 5, dadurch gekennzeichnet, daß man die α-Sulfofettsäurealkylester mit einer 30 bis 70 gew.-%igen wässrigen Lösung von Wasserstoffperoxid und festem Alkalimetallcarbonat versetzt und dabei ein Gewichtsverhältnis Ester : $H_2O_2$ von 1 : 0,03 bis 1 : 0,05 und ein Molverhältnis Ester : Alkalimetallcarbonat von 1 : 0,5 bis 1 : 0,65 einstellt.

7. Verfahren nach Ansprüchen 1 bis 5, dadurch gekennzeichnet, daß man die α-Sulfofettsäurealkylester mit einer 30 bis 70 gew.-%igen wässrigen Lösung von $H_2O_2$, festem Alkalimetallcarbonat und Alkalimetallhydroxid versetzt und dabei ein Gewichtsverhältnis Ester : $H_2O_2$ von 1 : 0,03 bis 1 : 0,05, ein Molverhältnis Ester : Alkalimetallcarbonat von 1 : 0,5 bis 1 : 0,525 und ein Molverhältnis Ester : Alkalimetallhydroxid von 1 : 0,03 bis 1 : 0,08 einstellt.

8. Verfahren nach Ansprüchen 6 und 7, dadurch gekennzeichnet, daß man als Alkalimetallcarbonat Natriumcarbonat verwendet.

9. Verfahren nach Anspruch 7, dadurch gekennzeichnet, daß man als Alkalimetallhydroxid Natriumhydroxid verwendet.

10. Verfahren nach Ansprüchen I bis 9, dadurch gekennzeichnet, daß man die Bleich- und Neutralisationsreaktion bei einer Temperatur im Bereich von 50 bis 80°C durchführt.

11. Verfahren nach Ansprüchen 1 bis 10, dadurch gekennzeichnet, daß man die Entgasung bei einem Druck von 0,2 bis 0,5 bar durchführt.

12. Verfahren nach Ansprüchen 1 bis 11, dadurch gekennzeichnet, daß man die Restentgasung und Restentwässerung bei einem Druck von 15 bis 100 mbar durchführt.

13. Verfahren nach Ansprüchen 1 bis 12, dadurch gekennzeichnet, daß man die Entgasung und/oder Restentgasung/Restentwässerung unter mechanischer Bearbeitung, bevorzugt unter Rühren oder Kneten, durchführt.

## Claims

1. A process for the production of solid, substantially anhydrous alkali metal salts of α-sulfofatty acid alkyl esters by oxidative bleaching and neutralization of α-sulfofatty acid alkyl esters containing from 8 to 22 carbon atoms in the fatty acid chain and from 1 to 6 carbon atoms in the ester alkyl radical, characterized in that

(a) in an apparatus suitable for the processing of pasteform products, the solid or molten α-sulfofatty acid alkyl esters are mixed simultaneously with an aqueous solution of hydrogen peroxide or an $H_2O_2$-yielding compound and with solid alkali metal carbonate at a temperature of from 20 to 80°C, optionally in the presence of aqueous alkali metal hydroxide, the ratio by weight of ester to $H_2O_2$ being from 1:0.01 to 1:0.06 and the molar ratio of ester to alkali metal carbonate being in the range of from 1:0.5 to 1:0.75,

(b) the foam formed through the release of $CO_2$ is mechanically destroyed under a pressure of from 0.2 to 1.0 bar and at a temperature of from 50 to 70°C,

(c) the product formed is residually degassed and residually dehydrated with mechanical agitation under a pressure of from 15 to 100 mbar and at a temperature of from 50 to 80°C and

(d) the solids formed are made up by known methods.

2. A process as claimed in claim 1, characterized in that a stirred tank, a kneader or a screw mixer is used as the apparatus suitable for processing paste-form products.

3. A process as claimed in claims 1 and 3, characterized in that molten α-sulfofatty acid alkyl esters are used.

4. A process as claimed in claims 1 and 2, characterized in that α-sulfofatty acid alkyl esters containing from 12 to 18 carbon atoms in the fatty acid chain are used.

5. A process as claimed in claims 1 and 2, characterized in that α-sulfofatty acid alkyl esters containing from 1 to 3 carbon atoms in the ester alkyl radical, preferably α-sulfofatty acid methyl esters, are used.

6. A process as claimed in claims 1 to 5, characterized in that a 30 to 70% by weight aqueous solution of hydrogen peroxide and solid alkali metal carbonate are added to the α-sulfofatty acid alkyl esters and a ratio by weight of ester to $H_2O_2$ of from 1:0.03 to 1:0.05 and a molar ratio of ester to alkali metal carbonate of from 1:0.5 to 1:0.65 are adjusted.

7. A process as claimed in claims 1 to 5, characterized in that a 30 to 70% by weight aqueous solution of $H_2O_2$, solid alkali metal carbonate and alkali metal hydroxide are added to the α-sulfofatty acid alkyl esters and a ratio by weight of ester to $H_2O_2$ of from 1:0.03 to 1:0.05, a molar ratio of ester to alkali metal carbonate of from 1:0.5 to 1:0.525 and a molar ratio of ester to alkali metal hydroxide of from 1:0.03 to 1:0.08 are adjusted.

8. A process as claimed in claims 6 and 7, characterized in that sodium carbonate is used as the alkali metal carbonate.

9. A process as claimed in claim 7, characterized in that sodium hydroxide is used as the alkali metal hydroxide.

10. A process as claimed in claims 1 to 9, characterized in that the bleaching and neutralization reaction is carried out at a temperature of from 50 to 80°C.

11. A process as claimed in claims 1 to 10, characterized in that the degassing is carried out under a pressure of from 0.2 to 0.5 bar.

12. A process as claimed in claims 1 to 11, characterized in that the residual degassing and residual dehydration are carried out under a pressure of from 15 to 100 mbar.

13. A process as claimed in claims 1 to 12, characterized in that the degassing and/or residual degassing/residual dehydration is/are carried out with mechanical agitation, preferably with stirring or kneading.

## Revendications

1. Procédé de fabrication de sels de métaux alcalins solides et presque anhydres d'esters alkyliques d'acides gras α-sulfonés par blanchiment par oxydation et neutralisation d'esters alkyliques d'acides gras α-sulfonés comportant 8 à 22 atomes de carbone dans la chaîne d'acide gras et 1 à 6 atomes de carbone dans le résidu alkyle de l'ester, caractérisé en ce que

(a) l'on mélange dans une installation appropriée au traitement des produits pâteux les esters alkyliques d'acides gras α-sulfonés solides ou en fusion, à des températures situées dans l'intervalle de 20 à 80°C, simultanément avec une solution aqueuse d'eau oxygénée ou d'un composé fournissant de l'$H_2O_2$ et avec un carbonate de métal alcalin solide, éventuellement en présence d'hydroxyde de métal alcalin en solution aqueuse, le rapport pondéral entre l'ester et l'eau oxygénée étant compris dans la plage de 1:0,01 à 0,06 et le rapport molaire entre l'ester et le carbonate de métal alcalin se situant dans l'intervalle de 1:0,5 à 1:0,75,

(b) l'on détruit mécaniquement la mousse produite par le dégagement de $CO_2$, sous une pression de 0,2 à 1,0 bar et à une température de 50 à 70°C,

(c) l'on élimine du produit formé l'eau et les gaz résiduels, sous agitation mécanique, à une pression de 15 à 100 mbar et à une température de 50 à 80°C,

(d) l'on prépare ou traite les matières solides obtenues selon des procédés connus.

2. Procédé selon la revendication 1, caractérisé en ce que l'on utilise, comme installation convenant pour le traitement des produits pâteux, une chaudière à agitation, un malaxeur ou un mélangeur à vis.

3. Procédé selon les revendications 1 et 2, caractérisé en ce que l'on met en œuvre un ester alkylique d'acide gras α-sulfoné en fusion.

4. Procédé selon les revendications 1 et 2, caractérisé en ce que l'on met en œuvre un ester alkylique d'acide gras α-sulfoné comportant 12 à 18 atomes de carbone dans la chaîne d'acide gras.

5. Procédé selon les revendications 1 et 2, caractérisé en ce que l'on met en œuvre un ester alkylique d'aoide gras α-sulfoné comportant 1 à 3 atomes de carbone dans le résidu alkyle de l'ester, de préférence un ester méthylique d'acide gras α-sulfoné.

6. Procédé selon les revendications 1 à 5, caractérisé en ce que l'on fait réagir l'ester alkylique d'acide gras α-sulfoné avec 30 à 70% en poids d'une solution aqueuse d'eau oxygénée et avec un carbonate de métal alcalin solide, le rapport pondéral entre l'ester et l'eau oxygénée étant compris dans l'intervalle de 1:0,03 à 1:0,05; et le rapport molaire entre l'ester et le carbonate de métal alcalin étant situé dans la plage de 1:0,5 à 1:0,65.

7. Procédé selon les revendications 1 à 5, caractérisé en ce que l'on fait réagir l'ester alkylique d'acide gras α-sulfoné avec 30 à 70% en poids d'une solution aqueuse d'eau oxygénée, avec un carbonate de métal alcalin solide et avec un hydroxyde de métal alcalin, le rapport pondéral entre l'ester et l'eau oxygénée étant compris dans l'intervalle de 1:0,03 à 1:0,05, le rapport molaire entre l'ester et le carbonate de métal alcalin étant situé dans la plage de 1:05 à 1:0, 525, et le rapport molaire entre l'ester et l'hydroxyde de métal alcalin étant compris dans l'intervalle de 1:0,03 à 1:0,08.

8. Procédé selon les revendications 6 et 7, caractérisé en ce que l'on utilise comme carbonate de métal alcalin, du carbonate de sodium.

9. Procédé selon la revendication 7, caractérisé en ce que l'on utilise comme hydroxyde de métal alcalin, de l'hydroxyde de sodium;

10. Procédé selon les revendications 1 à 9, caractérisé en ce que la réaction de blanchiment et de neutralisation se déroule à une température comprise dans l'intervalle de 50 à 80°C.

11. Procédé selon les revendications 1 à 10, caractérisé en ce que le dégazage est effectué à une pression de 0,2 à 0,5 bar.

12. Procédé selon les revendioations 1 à 11, caractérisé en ce que le dégazage et la déshydratation résiduels sont réalisés à une pression de 15 à 100 mbar.

13. Procédé selon les revendications 1 à 12, caractérisé en ce que le dégazage et/ou le dégazage/déshydratation résiduels sont effectués sous action mécanique, de préférence sous agitation ou malaxage.